Europäisches Patentamt

European Patent Office

Office européen des brevets

(19)

(11) Veröffentlichungsnummer: **0 000 885**
**B1**

(12) **EUROPÄISCHE PATENTSCHRIFT**

(45) Veröffentlichungstag der Patentschrift: **10.06.81**

(21) Anmeldenummer: **78100596.2**

(22) Anmeldetag: **07.08.78**

(51) Int. Cl.³: **C 07 C 49/597,**
**C 07 C 45/67 //C11B9/00**

(54) Verfahren zur Herstellung eines Cyclopentendions.

(30) Priorität: **12.08.77 CH 9910/77**

(43) Veröffentlichungstag der Anmeldung:
**07.03.79 Patentblatt 79/5**

(45) Bekanntmachung des Hinweises auf die Patenterteilung:
**10.06.81 Patentblatt 81/23**

(84) Benannte Vertragsstaaten:
**BE CH DE FR GB NL**

(56) Entgegenhaltungen:
**CHEMISCHE BERICHTE, Band 100,**
**September 1967 Weinheim**
**H. STETTER und H.—J. SANDHAGEN**
**"über den Verlauf der Dehydrobro-**
**mierung bei 4-Brom-2.2-dimethyl-**
**cyclohexandion-(1.3)",**
**Seiten 2837—2841**

(73) Patentinhaber: **F. HOFFMANN-LA ROCHE & CO.**
**Aktiengesellschaft**
**CH-4002 Basel (CH)**

(72) Erfinder: **Brenner, Wolf, Dr.**
**Poolstrasse 17**
**CH-4414 Füllinsdorf (CH)**

(74) Vertreter: **Lederer, Franz, Dr. et al,**
**Patentanwälte Lederer, Meyer Lucile-Grahn-**
**Strasse 22**
**D-8000 München 80 (DE)**

### Verfahren zur Herstellung eines Cyclopentendions

Die vorliegende Erfindung betrifft ein Verfahren zur Herstellung eines Cyclopentendions, nämlich von 2,2,4-Trimethyl-cyclopent-4-en-1,3-dion.

Es handelt sich bei dieser Verbindung um eine bekannte Substanz, welche gewisse olfaktorische Eigenschaften aufweist und ausserdem als Zwischenprodukt in verschiedenen Synthesen verwendbar ist.

Bisher war diese Verbindung nur auf umständlichem Wege, beispielsweise über das 3,3-Dimethyl-bicyclo[3.1.0.]hexandion(2,4) und in niedrigen Ausbeuten zugänglich ("Chemische Berichte" 1967, Seiten 2837—2841). Das erfindungsgemasse Verfahren, welches eine leichte Zugänglichkeit dieser Verbindung in hohen Ausbeuten ermöglicht, ist dadurch gekennzeichnet, dass man 3,5,5-Trimethyl-cyclohex-2-en-1,4-dion mit Sauerstoff oder einem Sauerstoff enthaltenden Gas in Gegenwart eines Katalysators auf der Basis von Mangan, Kobalt oder Kupfer oxydiert.

Als Katalysator wird hiebei zweckmässig ein Salz von Mangan, Kobalt oder Kupfer mit einer schwachen Säure, beispielsweise einer niederen Alkancarbonsäure, wie Ameisensäure, Essigsäure oder Propion säure verwendet.

Gemäss einer anderen, bevorzugten Ausführung des erfindungsgemässen Verfahrens wird als Katalysator ein Mangan-, Kobalt- oder Kupfersalz eines Enols, beispielsweise ein Acetylacetonat dieser Metalle verwendet.

Eine besonders bevorzugte Ausführungsform besteht darin, dass man als Katalysator Manganacetat oder Manganacetylacetonat verwendet.

Es hat sich als vorteilhaft erwisen, die Oxydation in Gegenwart von Pyridin oder einem Homologen von Pyridin, also einem niederalkyl-substituierten Pyridin, wie Collidin, Picolin, Lutidin oder 2-Methyl-5-äthyl-pyridin durchzuführen.

Die erfindungsgemässe Oxydation wird zweckmässig in einem inerten organischen Lösungsmittel durchgeführt, welches den Katalysator wenigstens, teilweise löst.

Beispiele solcher Lösungsmittel sind aromatische Kohlenwasserstoffe, wie Benzol, Toluol oder Xylol, Aether, insbesondere Diniederalkyl-äther wie Diäthyläther, oder Pyridin bzw. ein niederalkyl-substituiertes Pyridin, wobei in letzterem Falle dieses Lösungsmittel gleichzeitig die Funktion eines Co-Katalysators ausübt.

Die erfindungsgemässe Oxydation kann zweckmässig in einem Temperaturbereich zwischen etwa 40° und 110°C durchgeführt werden, insbesondere zwischen etwa 55° und etwa 70°C. Ein ganz besonders bevorzugter Temperaturbereich liegt zwischen 55 und 65°C.

Die Oxydation kann mit Sauerstoff oder einem sauerstoffhaltigen Gas durchgeführt werden. Wenn man Luft als Oxydationsmittel verwendet, verläuft die Reaktion etwa halb so schnell wie bei der Verwendung von reinem Sauerstoff.

### Beispiel

In einem 4-Liter-Kolben mit Rührer, Rückflusskühler, Gaseinleitrohr und Thermometer werden 220,5 g Mangan(II)acetat $\times$ 4H$_2$O in 1800 ml Pyridin gelöst. Bei 60°C unter Sauerstoffbegasung und intensivem Rühren werden 1368 g Ketoisophoron zugegeben. 3—4 Minuten danach findet eine schwache exotherme Reaktion statt. Gleichzeitig nimmt das Reaktionsgemisch langsam Sauerstoff auf.

Nach 20 Stunden Reaktionszeit sind etwa 73% des Ketoisophorons umgesetzt. Das Reaktionsgemisch wird destilliert (Kp. = 30—180°C/0,1 Torr).

Kondensat = 2937,5 g  
Rückstand = 143,8 g (nach Abzug des Katalysators)

Das Kondensat wird an einer 1,5-m-Kolonne mit Fenskering-Füllung fraktioniert.
Man erhält 602 g 2,2,4-Trimethyl-4-cyclopenten-1,3-dion.

### Patentansprüche

1. Verfahren zur Herstellung von 2,2,4-Trimethyl-cyclopent-4-en-1,3-dion, dadurch gekennzeichnet, dass man 3,5,5-Trimethylcyclohex-2-en-1,4,-dion mit Sauerstoff oder einem Sauerstoff enthaltenden Gas in Gegenwart eines Katalysators auf der Basis von Mangan, Kobalt oder Kupfer oxydiert.

2. Verfahren nach Anspruch 1, dadurch gekennzeichnet, dass man als Katalysator ein Salz von Mangan, Kobalt oder Kupfer mit einer schwachen Säure, beispielsweise einer niederen Alkancarbonsäure, wie Ameisensäure, Essigsäure oder Propionsäure verwendet.

3. Verfahren nach Anspruch 1, dadurch gekennzeichnet, dass man als Katalysator ein Mangan-, Kobalt- oder Kupfersalz eines Enols, beispielsweise ein Acetylacetonat, verwendet.

4. Verfahren nach Anspruch 1,2 oder 3, dadurch gekennzeichnet, dass man als Katalysator Manganacetat oder Manganacetylacetonat verwendet.

5. Verfahren nach einem der Ansprüche 1—4, dadurch gekennzeichnet, dass man die Oxydation in Gegenwart von Pyridin oder einem niederalkyl-substituierten Pyridin, wie Collidin, Picolin oder Lutidin, durchführt.

6. Verfahren nach einem der Ansprüche 1—5, dadurch gekennzeichnet, dass man die Oxydation in einem inerten organischen Lösungsmittel, welches den Katalysator wenigstens teilweise löst, durchführt.

7. Verfahren nach Anspruch 6, dadurch gekennzeichnet, dass man als Lösungsmittel einen aromatischen Kohlenwasserstoff, wie Benzol, Toluol oder Xylol, einen Aether, insbesondere einen Di-niederalkyl-äther wie Diäthyläther, oder Pyridin bzw. ein niederalkyl-substituiertes Pyridin verwendet.

8. Verfahren nach einem der Ansprüche 1—7, dadurch gekennzeichnet, dass man die Oxydation bei einer Temperatur zwischen etwa 40° und 110°C, insbesondere zwischen 55° und 70°C, durchführt.

## Claims

1. Process for the manufacture of 2,2,4-trimethyl-cyclopent-4-ene-1,3-dione, characterised in that 3,5,5-trimethyl-cyclohex-2-ene-1,4-dione is oxidised with oxygen or an oxygen-containing gas in the presence of a catalyst based on manganese, cobalt or copper.

2. Process according to claim 1, characterised in that a salt of manganese, cobalt or copper with a weak acid, for example a lower alkanecarboxylic acid, such as formic acid, acetic acid or propionic acid, is used as the catalyst.

3. Process according to claim 1, characterised in that a manganese, cobalt or copper salt of an enol, for example an acetylacetonate, is used as the catalyst.

4. Process according to claim 1, 2 or 3, characterised in that manganese acetate or manganese acetylacetonate is used as the catalyst.

5. Process according to one of claims 1—4, characterised in that the oxidation is carried out in the presence of pyridine or a lower alkyl-substituted pyridine, such as collidine, picoline or lutidine.

6. Process according to one of claims 1—5, characterised in that the oxidation is carried out in an inert organic solvent which at least partially dissolves the catalyst.

7. Process according to claim 6, characterised in that an aromatic hydrocarbon, such as benzene, toluene or xylene, an ether, especially a di-lower alkyl ether, such as diethyl ether, or pyridine or a lower alkyl-substituted pyridine is used as the solvent.

8. Process according to one of claims 1—7, characterised in that the oxidation is carried out at a temperature between about 40° and 110°C, especially between 55° and 70°C.

## Revendications

1. Procédé pour la préparation de la 2,2,4-triméthyl-cyclopent-4-ène-1,3-dione, caractérisé par le fait qu'on oxyde la 3,5,5-triméthyl-cyclohex-2-ène-1,4-dione avec de l'oxygène ou un gaz en contenant, en présence d'un catalyseur à base de manganèse, de cobalt, ou de cuivre.

2. Procédé suivant la revendication 1, caractérisé par le fait qu'on utilise comme catalyseur un sel de manganèse de cobalt ou de cuivre et d'un acide faible, par exemple d'un acide alcanecarboxylique inférieur tel que l'acide formique, l'acide acétique ou l'acide propionique.

3. Procédé suivant la revendication 1, caractérisé par le fait qu'on utilise comme catalyseur un sel de manganèse, de cobalt ou de cuivre d'un énol, par exemple un acétylacétonate.

4. Procédé suivant l'une des revendications 1, 2 et 3, caractérisé par le fait qu'on utilise comme catalyseur de l'acétete de manganèse ou de l'acétylacétonate de manganèse.

5. Procédé suivant l'une des revendications 1 à 4, caractérisé par le fait qu'on effectue l'oxydation en présence de pyridine ou d'une alkylpyridine inférieure telle que la collidine, la picoline ou la lutidine.

6. Procédé suivant l'un des revendications 1 à 5, caractérisé par le fait qu'on effectue l'oxydation dans un solvant organique inerte qui dissout au moins partiellement le catalyseur.

7. Procédé suivant la revendication 6, caractérisé par le fait qu'on utilise comme solvant un hydrocarbure aromatique tel quedubenzène, du toluène ou du xylène, un éther, en particulier un éther alkylique inférieur tel que l'éther éthylique, ou de la pyridine ou une alkylpyridine inférieure.

8. Procédé suivant l'une des revendications 1 à 7, caractérisé par le fait qu'on effectue l'oxydation à une température comprise entre environ 40° et 110°C, en particulier entre 55° et 70°C.